# EUROPÄISCHE PATENTSCHRIFT

(11) **EP 1 019 430 B1**
(45) Veröffentlichungstag und Bekanntmachung des Hinweises auf die Patenterteilung: **11.07.2007**
(21) Anmeldenummer: 98952670.2
(22) Anmeldetag: 29.09.1998
(51) Int. Cl.: C07H 21/00

(54) **Verfahren zur Isolierung einer Nukleinsäure**
Method for isolating a nucleic acid
Procédé pour l'isolation d'un acide nucleique

(30) Priorität: 01.10.1997 DE 19743518
(43) Veröffentlichungstag der Anmeldung: 19.07.2000
(60) Teilanmeldung: 07003878.1
(73) Patentinhaber: Roche Diagnostics GmbH, 68305 Mannheim (DE)
(72) Erfinder: KLEIBER, Jörg, D-82377 Penzberg (DE); MARKERT-HAHN, Christine, D-82377 Penzberg (DE); HARTTIG, Herbert, D-67122 Altrip (DE)
(86) Internationale Anmeldenummer: PCT/EP1998/006196
(87) Internationale Veröffentlichungsnummer: WO 1999/016781

(56) Entgegenhaltungen:
- EP-A- 0 757 106
- WO-A-88/06633
- WO-A-96/41811
- BOOM R ET AL: "RAPID AND SIMPLE METHOD FOR PURIFICATION OF NUCLEIC ACIDS" JOURNAL OF CLINICAL MICROBIOLOGY, Bd. 28, Nr. 3, März 1990 (1990-03), Seiten 495-503, XP002911989 ISSN: 0095-1137 in der Anmeldung erwähnt

## Beschreibung

Die Erfindung betrifft ein Verfahren zur Vorbereitung biologischer Proben für den anschließenden Nachweis eines Analyten, insbesondere einer Nukleinsäure, in dieser Probe. Weiterhin werden Reagenzienkits bereitgestellt.

Bei einem Verfahren zum Nachweis eines Analyten in einer biologischen Probe werden an die Probenvorbereitung oft besondere Anforderungen gestellt. Zum einen ist der Analyt oft in sehr geringer Konzentration vorhanden und zum anderen finden sich oft viele andere Substanzen in der Probe, die die Isolierung bzw. Bestimmung des Analyten beeinträchtigen können.

WO 36/41811 offenbart ein Verfahren zur Isolierung eines Analyten, insbesondere einer Nukleinsäure, aus einer biologischen Probe, wobei die Probe, die den Analyten in einer Flüssigkeit enthält, mit magnetischen Partikeln, die eine äußere Glasoberfläche, die im wesentlichen porenfrei ist oder Poren eines Durchmessers von < 10 nm aufweist, in Kontakt gebracht wird, unter Bedingungen, bei denen der Analyt an die Partikeloberfläche bindet, und der gebundene Analyt von der Probenflüssigkeit abgetrennt wird. Die magnetischen Partikel werden hier in fester Form und nicht in Form einer Suspension eingesetzt. Die Glasoberfläche besteht aus einem Borosilikatglas. Das in WO 96/46811 beschriebene Verfahren eignet sich sehr gut zur Aufreinigung eines Analyten aus einer biologischen Probe. Es ist jedoch nicht ohne weiteres für eine automatisierte Probenvorbereitung einsetzbar.

Boom et al. (J. Clin. Microbiol. 28 (1990), 495-503) beschreiben ebenfalls ein Protokoll für Aufreinigung von Nukleinsäuren aus einer biologischen Probe unter Verwendung größenfraktionierter Siliciumoxidteilchen. Dieses Verfahren ist jedoch umständlich, nicht für eine Automatisierung geeignet und enthält darüber hinaus die Gefahr von Verschleppungen.

Bei einer in EP-A-0 757 106 beschriebenen Methode zur Extraktion von Nukleinsäuren wird eine Probe lysiert, die in der Probe vorhandenen Nukleinsäuren an superparamagnetische Metallteilchen gebunden, diese mit einer Pipette aus dem Probengefäß entfernt und somit von den übrigen Probenbestandteilen abgetrennt. Dieses Verfahren hat den Nachteil, daß aufgrund der Notwendigkeit, den Analyten mit einer Pipette aus der Probe zu entfernen, Verluste auftreten können. Darüber hinaus beinhaltet die Verwendung mehrerer Reaktionsgefäße die Gefahr von Verschleppungen und Kontaminationen.

Die der vorliegenden Erfindung zugrundeliegende Aufgabe bestand somit darin, ein neues Probenvorbereitungsverfahren bereitzustellen, bei dem die Nachteile des Standes der Technik mindestens teilweise beseitigt sind. Insbesondere soll das neue Verfahren automatisierbar sein und ein möglichst einfaches Temperaturprofil aufweisen.

Diese Aufgabe wird gelöst durch Verfahren zur Isolierung eines Analyten aus einer biologischen Probe, umfassend die Schritte:
(a) Aufschließen der Probe in einem Reaktionsgefäß,
(b) Zugeben von magnetischen Glaspartikel in Form einer alkoholischen Suspension.
(c) Inkubieren unter Bedingungen, bei denen der Analyt an die Glaspartikel bindet,
(d) Entfernen nichtgebundener Probenbestandteile aus dem Reaktionsgefäß,
(e) Inkubieren unter Bedingungen, bei denen der Analyt von den Glaspartikeln eluiert wird, und
(f) Abtrennen des Eluats von den Glaspartikeln,
wobei viele oder alle Schritte bei im wesentlichen der gleichen Temperatur durchgeführt werden, die vorzugsweise im Bereich von Raumtemperatur bis 40°C liegt.

Das erfindungsgemäße Verfahren beruht auf der selektiven Bindung von Analyten an eine feste Adsorptionsmatrix in Gegenwart eines Probenaufschlußpuffers, wobei der Analyt, bei dem es sich vorzugsweise um eine Nukleinsäure wie DNA, z.B. chromosomale DNA, fragmentierte chromosomale DNA, Plasmid-DNA, virale DNA etc., oder RNA, z.B. mRNA, tRNA, rRNA oder virale. RNA etc. handelt, von Verunreinigungen der Probe wie etwa Proteinen oder Zelltrümmern abgetrennt wird. Die Probe kann eine beliebige biologische Probe sein, z.B. eine Körperflüssigkeit wie Blut, Plasma, Urin etc, eine Gewebeprobe, eine Probe von kultivierten Zellen oder ähnliches.

Die beim erfindungsgemäßen Verfahren verwendete Adsorptionsmatrix ist in der Lage, eine unter den Reaktionsbedingungen weitgehend selektive Bindung des Analyten zu gewährleisten. Erfindungsgemäß verwendet man magnetische Glaspartikel, insbesondere die in WO 96/41811 beschriebenen magnetischen Partikel mit einer äußeren Glasoberfläche, die im wesentlichen porenfrei ist oder Poren eines Durchmessers von weniger als 10 nm aufweist. Besonders bevorzugt sind ferromagnetische Partikel, die eine Korngröße zwischen 10 und 60 µm aufweisen. Solche Partikel können beispielsweise einen Kern aus Glimmer und darauf immobilisierten Magnetpartikeln enthalten, der von der Glasschicht umschlossen ist. Während in WO 96/4181 die magnetischen Partikel in fester Form, z.B. als Tabletten oder Pulver, in den jeweils verwendeten Reaktionsgefäßen vorgelegt werden, setzt man die magnetischen Partikeln erfindungsgemäß in Form einer alkoholischer Suspension ein. B sonders geeignet haben sich alkoholische Suspensionen mit einer Konzentration von etwa 5 bis 20 mg/ml erwiesen. Überraschenderweise wurde festgestellt, daß trotz der hohen spezifischen Dichte der magnetischen Glaspartikel die Suspension mit hoher Reproduzierbarkeit aus einem Vorratsbehälter abgezogen werden kann, wodurch eine Automatisierbarkeit der Verfahrensführung ermöglicht wird.

Obwohl beim erfindungsgemäßen Verfahren die in WO 96/41811 beschriebenen Glaspartikel gute Resultate zeigen, werden besonders gute Ergebnisse mit Glaspartikeln erhalten, deren Glasphase folgende Metalloxide umfasst. SiO₂, B₂O₃, Alkalimetalloxid, z.B. K₂O oder/und Na₂O sowie gegebenenfalls Al₂O₃ und ein Erdalkalimetalloxid z.B. CaO. Die Anteile dieser Metalloxide sind vorzugsweise wie folgt: 50 bis 95 mol-% SiO₂, 0,2 bis 30 mol-% B₂O₃, 0 bis 10 mol-% Al₂O₃, 0 bis 20 mol-% Erdalkalimetalloxid und 0,2 bis 20 mol-% Alkalimetalloxid, wobei die Prozentangaben jeweils auf das Gesamtgewicht der Glasphase bezogen sind.

Für die Isolierung von RNA hat sich beispielsweise eine Glasphase, die SiO₂, B₂O₃, K₂O, Al₂O₃ und CaO enthält, als besonders geeignet erwiesen. Für die Isolierung von DNA hat sich eine Glasphase, die SiO₂, B₂O₃ und Na₂O enthält, als besonders geeignet erwiesen.

Die Adsorptionsmatrix wird beim erfindungsgemäßen Verfahren vorzugsweise in einer Menge zugegeben, welche der minimalen, zur quantitativen Bindung des in der Probe vorhandenen Analyten, insbesondere einer Nukleinsäure, benötigten Menge entspricht oder etwas größer, vorzugsweise um höchstens 50% und besonders bevorzugt um höchstens 20% über dieser Menge liegt. Die zu erwartende Nukleinsäuremenge in verschiedenen Arten von Proben kann - sofern sie nicht bereits bekannt ist - vorab durch übliche Techniken, z.B. Phenol/ Chloroform-Extraktion und anschließende Messung der optischen Dichte ermittelt werden.

Schritt (a) des erfindungsgemäßen Verfahrens umfaßt das Aufschließen der Probe in einem Reaktionsgefäß. Dieser Aufschluß erfolgt im Allgemeinen durch Lyse der in der Probe vorhandene Zellen unter denaturierenden Bedingungn ,z.B. durch Zugabe einer Protease und eines Denaturierungspuffers. Als Proteinase wird vorzugsweise Proteinase K, Pronase, Elastase oder/und Lysozym verwendet. Besonders bevorzugt ist die Verwendung von Proteinase K.

Der Proteaseverdau erfolgt in einem Denaturierungspuffer, der eine chaotrope Verbindung, z.B. Harnstoff oder Harnstoffderivate, vorzugsweise ein chaotropes Salz, besonders bevorzugt ein Guanidiniumsalz wie etwa Guanidiniumhydrochlorid (insbesondere zur Isolierung von DNA) oder Guanidiniumthiocyanat (insbesondere zur Isolierung von RNA) oder ein Perchlorat oder Iodid enthält. Für Guanidiniumsalze sind Konzentrationen im Bereich von 1 bis 3 mol/l bevorzugt.

Im Gegensatz zu der in WO 96/41811 beschriebenen Methode zur Probenvorbereitung erfolgt die Zugabe der festen Adsorptionsmatrix erst nach Aufschluß der Probe. Durch diese Verfahrensführung erreicht man eine signifikant geringere unspezifische Bindung von unerwünschten Probenbestandteilen, z.B. Proteinen, an der Adsorptionsmatrix.

Gemäß Schritt (c) erfolgt die selektive Bindung des Analyten an die Adsorptionsmatrix durch Inkubation im Aufschlußpuffer vorzugsweise unter chaotropen Bedingungen.

Schritt (d) des erfindungsgemäßen Verfahrens umfaßt das Abtrennen nicht gebundener Probenbestandteile von der Adsorptionsmatrix. Vorzugsweise werden hierzu die nichtgebundenen Probenbestandteile aus dem Reaktiosgefäß entfernt. Dies kann durch gegebenenfalls mehrmaliges Zugeben und Entfernen eines Waschpuffers erfolgen, der vorzugsweise einen Gehalt von mindestens 50% (v/v) und besonders bevorzugt von mindestens 60% (v/v) eines mit Wasser mischbaren organischen Lösungsmittels wie etwa Ethanol, Propanol und Aceton enthält.

Die Schritte (c), (d) oder/und (e) des erfindungsgemäßen Verfahren erfolgen vorzugsweise unter kontinuierlichem oder intervallartigem Mischen (d.h. Phasen, während denen gemischt wird, wechseln sich mit Phasen ab, in denen sich das Reaktionsgefäß im Ruhen befindet) ohne Zusatz externer Mittel. Vorzugsweise erfolgt dieses Mischen durch Drehung des Reaktionsgefäßes um seine Längsachse mit mehrmaliger Umkehr der Drehrichtung. Besonders bevorzugt wird das Mischungsgefäß exakt um seine Längsachse gedreht und der Drehrichtungswechsel so durchgeführt, daß die Meniskusauslenkung der Flüssigkeit unter einer vorbestimmten Trennziffer bleibt. Solche Mischverfahren sind in WO91/15768 und EP-A-0 435 481 beschrieben.

Die Dauer für die Schritte (c) oder/und (e) beträgt vorzugsweise maximal 20 min und umfaßt ein kontinuierliches Mischen oder ein Intervallmischen in kurzen Zyklen, vorzugsweise in kurzen Zyklen von vorzugsweise maximal 2 Minuten. Besonders gute Ergebnisse wurden durch Intervallmischen in einem einminütigen Zyklus umfassend 20 sec Mischen und 40 sec Ruhen erhalten.

Bei Verwendung von magnetischen Partikeln als Adsorptionsmatrix kann die Zugabe von Flüssigkeiten in das Reaktionsgefäß bzw. das Absaugen von Flüssigkeiten daraus unter kontinuierlichem Mischen erfolgen, wobei die Partikel während des Absaugevorgangs durch Einschalten des Magneten im Reaktionsgefäß gehalten werden. Durch diese Mischtechnik kann das erfindungsgemäße Verfahren flexibel auf verschiedene Probenarten eigestellt werden. Darüber hinaus wird ständig für eine gleichmäßige Verteilung der magnetischen Partikel in der Flüssigphase gesorgt.

Schritt (e) des erfindungsgemäßen Verfahrens umfasst die Elution des Analyten von der Adsorptionsmatrix. Hierzu kann einerseits - wie aus dem Stand der Technik bekannt - ein von organischen Lösungsmitteln im wesentlichen freier Niedrigsalzpuffer verwendet werden. Überraschenderweise wurde jedoch festgestellt, daß der Elutionspuffer zusätzliche Reagenzien enthalten kann, wie etwa Enzyme, z.B. zur Manipulation von Nukleinsäuren verwendete Enzyme wie etwa RNasen, DNasen, Restriktionsendonukleasen, Ligasen, terminale Transferasen oder/und Polymerasen. Wenn der Analyt beispielsweise eine DNA ist, kann während der Elution eine DNase-freie RNase zugesetzt werden, um den Gehalt an unerwünschter RNA zu verringern. Andererseits kann - wenn der Analyt eine RNA ist - während der Elution eine RNase-freie DNase zugesetzt werden. Auf entsprechende Weise können auch andere Enzyme, wie etwa Restriktionsendonukleasen, etc. zugesetzt werden. Wenn die durch das erfindungsgemäße Verfahren isolierte Nukleinsäure nachfolgend einer Amplifikation unterzogen wird, kann während der Elution auch ein Nukleinsäureamplifikations-Mastermix, welcher den Amplifikationspuffer, Nukleotide, Primer, Polymerase und Puffersalze enthält, zugesetzt werden.

Schritt (f) des erfindungsgemäßen Verfahrens umfaßt das Abtrennen des Eluats von der Adsorptionsmatrix. Dieses Abtrennen kann auf übliche Weise erfolgen, z.B. durch Sedimentation, vorzugsweise aber durch magnetische Separation.

Die durch das erfindungsgemäße Verfahren isolierten Analyten können anschließend auf bekannte Weise weiterverarbeitet werden, im Fall von Nukleinsäuren, z.B. durch Amplifikation und nachfolgende Detektion, Detektion ohne vorhergehende Amplifikation oder Sequenzierung. Hierzu können Aliquots des Eluats selbst Bestimmungen unterschiedlicher Analyten zugeführt werden, z.B. verschiedene Viren, wie HIV, HCV und HBV.

Ein wichtiges Merkmal des erfindungsgemäßen Verfahrens ist, daß viele oder gegebenenfalls sogar alle Schritte bei im wesentlichen der gleichen Temperatur, d.h. innerhalb eines Temperaturbereichs von ± 2,5°C durchgeführt werden können. Vorzugsweise ist diese Temperatur im Bereich von Raumtemperatur bis 70°C, besonders vorzugt von Raumtemperatur bis 40°C, am meisten bevorzugt bei Raumtemperatur, d.h. ca. 18 bis 32°C. In einer bevorzugten Ausführungsform des erfindungsgemäßen Verfahrens werden zumindest die Schritte (c) der Adsorption und (d) des Waschens bei dieser Temperatur durchgeführt. Besonders bevorzugt werden auch andere Schritte, insbesondere die Schritte (a) des Aufschließens oder/und (e) der Elution bei dieser Temperatur durchgeführt. Für die Bestimmung von HIV in Blutproben kann beispielsweise die gesamte Probenvorbereitung bei einer einheitlichen Temperatur erfolgen. Gegebenenfalls kann nach Schritt (f) des erfindungsgemäßen Verfahrens zusätzlich ein Nachbehandlungsschritt bei erhöhter Temperatur erfolgen, wodurch bei bestimmten Analyten die Ausbeuten bei einer Amplifikation verbessert wird. Bei anderen Analyten kann es erforderlich sein, daß die Vorbehandlung oder/und die Elution bei einer erhöhte Temperatur erfolgen. Die erhöhte Temperatur liegt dabei vorzugsweise im Bereich von mehr als 40°C bis 95°C, z.b. ca. 70 °C.

Die Durchführung des erfindungsgemäßen Verfahrens erfolgt vorzugsweise in einer automatisierten Vorrichtung. Beispiele für solche Vorrichtungen sind nachfolgend beschrieben. Weiterhin ist bevorzugt, daß das erfindungsgemäße Verfahren zur Probenvorbereitung, zumindest die Schritte (a) bis (e) in einem einzigen Reaktionsgefäß durchgeführt werden, d.h. daß kein Transfer in ein anderes Reaktionsgefäß erfolgt. Dies hat eine erhebliche Vereinfachung des Verfahrens zur Folge und führt darüber hinaus zu einem verringertem Kontaminationsrisiko.

Noch ein weiterer Gegenstand der Erfindung ist ein Reagenzienkit, der insbesondere zur Durchführung des vorstehend beschriebenen Verfahrens geeignet ist, umfassend
(a) eine Protease,
(b) einen Probenaufschlußpuffer,
(c) einen Waschpuffer,
(d) einen Elutionspuffer und
(e) eine alkoholische Suspension von magnetischen Glaspartikeln.

Auch beschrieben, aber nicht beansprucht, ist eine Vorrichtung zur Isolierung eines Analyten aus einer biologischen Probe, umfassend:
- eine Aufnahmeeinrichtung für Reagenzien (2),
- eine erste Aufnahmeeinrichtung für Reaktionsgefäße zur Probenvorbereitung (3), die für eine Betriebstemperatur von ≤ 70°C, insbesondere ≤ 40°C eingerichtet ist,
- eine zweite Aufnahmeeinrichtung für Reaktionsgefäße (4a, 4b, 4c), die gegebenenfalls Kühl- oder/und Heizmittel enthält,
- und automatische Pipettiervorrichtungen.

Die Vorrichtung ist vorzugsweise so ausgestaltet, daß ein einziges Reaktionsgefäß zur Durchführung der 4 Hauptschritte der Probenvorbereitung, nämlich Aufschluß einer Probe bzw. Lyse, Adsorption des freigesetzten Analyten, z.B. einer Nukleinsäure, an eine feste Adsorptionsmatrix, z.B. magnetische Glaspartikel, Waschen der Adsorptionsmatrix und Elution des Analyten von der Adsorptionsmatrix vorgesehen ist.

Die Vorrichtung ist so ausgestaltet, daß die erste Aufnahmeeinrichtung zur Aufnahme der Reaktionsgefäße für die Probenvorbereitung zumindest für die Adsorption des Analyten an die fest Adsorptionsmatrix und für das Waschen der Adsorptionsmatrix vorgesehen ist. In einer bevorzugten Ausführungsform ist die erste Aufnahmeeinrichtung weiterhin zum Aufschluß der Probe oder/und zur Elution des Analyten von der Adsorptionsmatrix vorgesehen. Die Reaktionsgefäße zur Probenvorbereitung haben ein Volumen von vorzugsweise mindestens 1 ml, z.B. 1-5 ml.

Die zweite Aufnahmeeinrichtung ist für Reaktionsgefäße zur Aufbewahrung oder/und Weiterverarbeitung des Analyten vorgesehen, z.B. PCR-Gefäße, die üblicherweise eine von den zur Probenvorbereitung verwendeten Reaktionsgefäßen verschiedene Form aufweisen. Die Reaktionsgefäße zur Aufbewahrung oder/und Weiterverarbeitung haben ein Volumen von vorzugsweise bis zu 500 µl, z.B. 50-200 µl. Darüber hinaus kann die zweite Aufnahmeeinrichtung Gefäße für Reagenzien enthalten, die zur Weiterverarbeitung der den Analyten enthaltenden Probe benötigt werden, z.B. einen PCR-Mastermix.

Die Vorrichtung kann so ausgestaltet sein, daß einer oder mehrere Schritte der Probenvorbereitung oder/und ein Nachbehandlungsschritt bei einer erhöhten Temperatur in der zweiten Aufnahmeeinrichtung erfolgen können. Hierzu kann die zweite Aufnahmeeinrichtung zur Aufnahme von Reaktionsgefäßen für zumindest einen Behandlungsschritt vorgesehen sein, der ausgewählt ist aus dem Aufschluß der Probe, der Elution der Probe von der Adsorptionsmatrix und einem Nachbehandlungsschritt nach der Elution.

Die erste Aufnahmeeinrichtung umfaßt vorzugsweise Mittel zur magnetischen Separation. Weiterhin ist bevorzugt, daß die erste Aufnahmeeinrichtung Mittel zum Mischen der Reaktionsgefäße, insbesondere durch Drehen um deren Längsachse umfaßt. Solche Mittel können gegebenenfalls auch für die zweite Aufnahmeeinrichtung vorgesehen sein.

Die Vorrichtung umfasst im allgemeinen automatische Pipettiereinrichtungen sowie gegebenenfalls Mittel zum Transport von Reaktionsgefäßen, z. B. zwischen erster und zweiter Aufnahmeeinrichtung. Außerdem kann eine Deckel-Öffnungs- und Schließeinheit integriert sein.

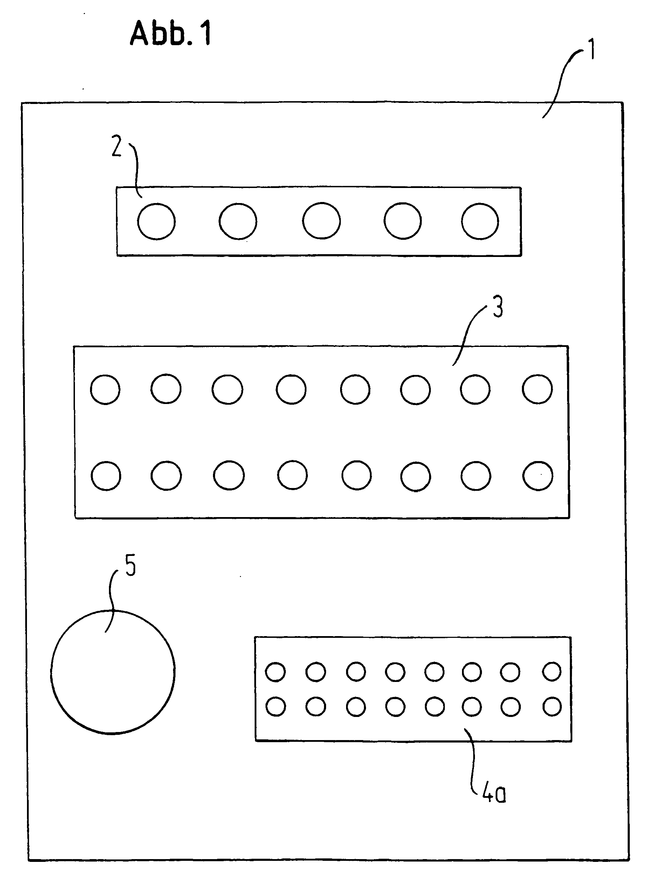

Im folgenden sind spezielle Ausführungsformen im Detail dargestellt. Bei der in Abbildung 1 gezeigten Ausführungsform enthält die Vorrichtung (1) eine Aufnahmeeinrichtung für Reagenzien (2), eine Aufnahmeeinrichtung für Reaktionsgefäße zur Probenvorbereitung (3) mit den Funktionen Mischen und Magnetseparation, die für eine Temperatur von vorzugsweise ≤ 40°C und besonders bevorzugt Raumtemperatur vorgesehen ist. Weiterhin enthält die Vorrichtung eine Aufnahmestation für weitere Reaktionsgefäße (4a), z.B. für PCR-Gefäße, die eine Temperatur von 4°C bis Raumtemperatur aufweist. Weiterhin enthält die Vorrichtung automatisierte Einrichtungen für die Pipettierung und Handhabung von Reaktionsgefäßen (5), die Bewegungen in X, Y und Z Richtung ermöglichen. Bei dieser Ausführungsform der erfindungsgemäßen Vorrichtung finden die vier Hauptschritte der Probenvorbereitung, nämlich Lyse, Adsorption, Waschen und Elution in der ersten Aufnahmeeinrichtung in einem einzigen Reaktionsgefäß statt. Die Lagerung von Eluaten und die Zugabe weiterer Reagenzien, z.B. PCR-Mastermix, erfolgt in der zweiten Aufnahmeeinrichtung. Zur Weiterverarbeitung, z.B. für eine nachfolgende PCR, werden die Gefäße zu einer entsprechenden Vorrichtung, z.B. einem Thermocycler (nicht gezeigt) transferiert.

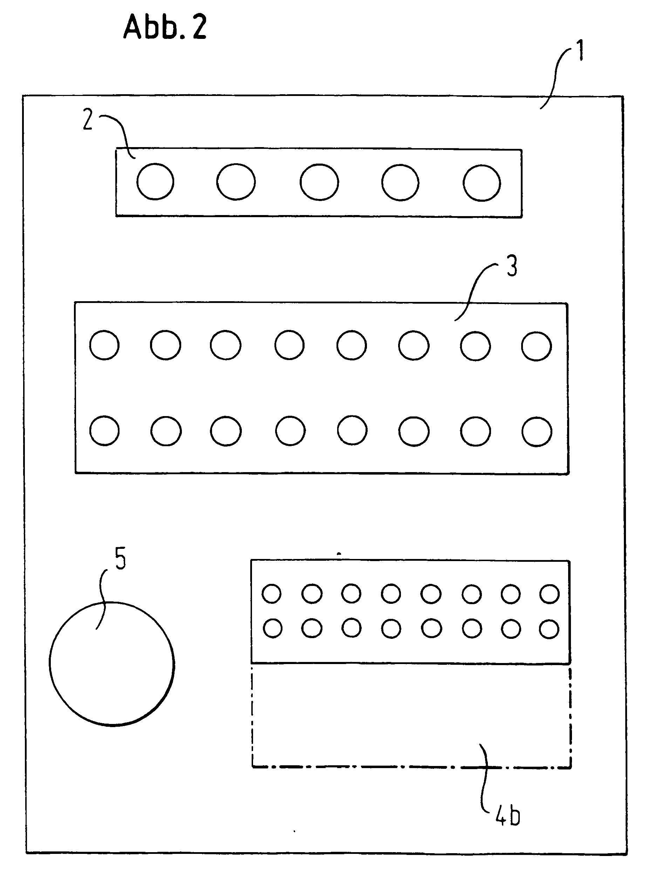

In der in Abbildung 2 gezeigten Ausführungsform enthält die Vorrichtung eine zweite Aufnahmeeinrichtung (4b), die zur Aufnahme von Weiterverarbeitungsreaktionsgefäßen, z.B PCR-Gefäßen, vorgesehen ist und die Einstellung einer Temperatur von 4°C (Kühlung des PCR-Mastermix) bis 95°C zum Erhitzen des Eluats nach der Elution von der Adsorptionsmatrix vorgesehen ist. Zur Vermeidung der Kondensatbildung am Deckel der PCR-Gefäße ist eine Deckelgegenheizung bevorzugt.

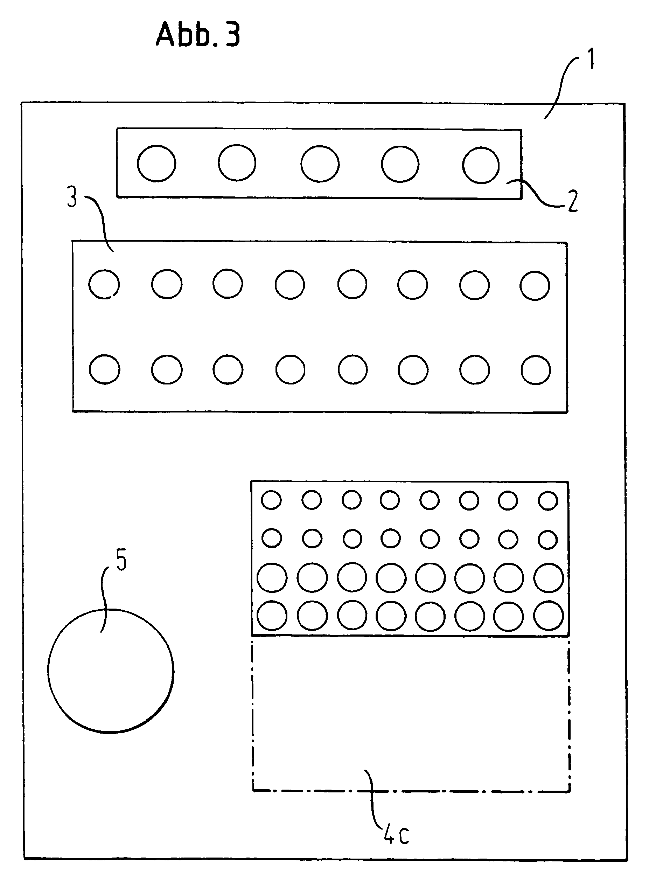

Gemäß der in Abbildung 3 dargestellten Ausführungsform der erfindungsgemäßen Vorrichtung ist eine zweite Aufnahmeeinrichtung für Reaktionsgefäße (4c) vorgesehen, welche zur Aufnahme von PCR-Gefäßen und Probenvorbereitungsgefäßen vorgesehen ist. In dieser zweiten Aufnahmeeinrichtung kann eine Kühlung, z.B. auf 4°C, und ein Aufheizen, z.B. auf 95°C, zum Erhitzen des Lysats oder/und des Eluats erfolgen. Auch hier ist zur Vermeidung der Kondensatbildung am Deckel von Reaktionsgefäßen eine Deckelgegenheizung bevorzugt.

Gemäß noch einer weiteren Ausführungsform der vorliegenden Erfindung (nicht gezeigt) ist die erste Aufnahmeeinrichtung zur Einstellung einer Temperatur im Bereich von ≤ 70°C vorgesehen. Die zweite Aufnahmeeinrichtung ist - wie in Abbildung 3 gezeigt - für das Kühlen und Heizen von Probenweiterverarbeitungs- und Probenvorbereitungsgefäßen geeignet.

Weiterhin wird die vorliegende Anmeldung durch die folgenden Figuren und Beispiele näher erläutert. Es zeigen:
- Abbildung 1: die schematische Darstellung einer ersten Ausführungsform der Vorrichtung,
- Abbildung 2: die schematische Darstellung einer zweiten Ausführungsform der Vorrichtung.
- Abbildung 3: die schematische Darstellung einer dritten Ausführungsform der Vorrichtung,
- Abbildung 4: das Ergebnis eines Chlamydien-Nachweises durch PCR mit manueller und semiautomatisierter Probenvorbereitung,
- Abbildung 5: das Ergebnis eines Chlamydien-Nachweises durch PCR mit semiautomatisierter Probenvorbereitung und unterschiedlichen Temperaturprofilen bei der Probenvorbereitung,
- Abbildung 6: das Ergebnis eines HIV-Nachweises durch PCR mit manueller Probenvorbereitung (Standardprotokoll) und semiautomatisierter Probenvorbereitung bei Raumtemperatur.

### Beispiele

### 1. Herstellung von magnetischen Glaspartikeln

Es wurden zwei verschiedene Sole verwendet. Die Herstellung der Sole erfolgte wie folgt:

### Sol 1: (SiO₂:B₂O₃:Na₂O=40:8:2)

Alkokolate der Oxide wurden in obigen Molverhältnissen analog der Vorgehensweise in Beispiel 1 und 2 von W096/4181 miteinander zu einer homogenen Phase verrührt. In Abweichung dazu wurde kein HCl eingesetzt.

Anschließend wurden 30 g Iriodin 600 Black Mica (Merk) in 100 ml Sol eingerührt.

### Sol 2: (SiO₂:B₂O₃:K₂O:Al₂O₃:CaO=76:15:5:2:2)

Alkokolate der Oxide wurden in obigen Molverhältnissen analog der Vorgehensweise in Beispiel 1 und 2 von WO96/41811 miteinander zu einer homogenen Phase verrührt. In Abweichung dazu wurde kein HCl eingesetzt.

Anschließend wurden 30 g Iriodin 600 Black Mica (Merk) in 100 ml Sol eingerührt.

Die Sole wurden anschließend einem Sprühtrocknungsvorgang unterzogen.

Das durch die Sprütrocknung erhaltene Pulver wurde einer Feinteilabtrennung durch Sedimentation, einer Temperaturbehandlung unter Stickstoffatmosphäre (60 l/h Volumenstrom) bei einer Aufheizgeschwindigkeit von 1 K/min unterzogen und 1 h einer Verdichtungstemperatur im Bereich von 600 bis 700°C für eine Stunde gehalten. Anschließend wurde der Ofen auf 300°C abgekühlt und bei dieser Temperatur für 1 h mit Sauerstoff gespült. Nach Abkühlung auf Raumtemperatur wurden die magnetischen Glaspartikel entnommen und zur Abtrennung des Grobanteils auf ein 50 µm-Sieb aufgegeben und gesiebt.

Die aus Sol 1 erhaltenen magnetischen Glaspartikel sind insbesondere zur Isolierung von DNA geeignet. Die aus Sol 2 erhaltenen Glaspartikel sind insbesondere zur Isolierung von RNA geeignet.

### 2. Standardprotokoll zur Probenvorbereitung für die Isolierung von Nukleinsäuren, z.B. DNA

Zur Isolierung von Nukleinsäuren aus biologischen Proben wie etwa Vollblut oder kultivierten Zellen ist das folgende Standardprotokoll geeignet. Die auf diese Weise erhaltenen Nukleinsäuren können direkt nach der Elution für eine Amplifikation durch PCR, eine Restriktionsspaltung oder einen Southernblot eingesetzt werden.

Der Reaktionskit enthält:
1. Bindepuffer (4,7 mol/l Guanidiniumhydrochlorid, 10mmol/l Harnstoff, 10 mmol/l Tris HCl, 20% Triton^{®} X-100, pH 5,7
2. lyophilisierte Proteinase K (zur Auflösung in H₂O auf eine Konzentration von 20 mg/ml)
3. Waschpuffer (56% (v/v) Ethanol, 20 mmol/NaCl, 10 mmol/l Tris HCl pH 7,5)
4. Elutionspuffer (10 mmol/l Tris pH 8,5)
5. magnetische Glaspartikel (MPG)
   a) Tabletten mit jeweils 7,5 mg der Glaspartikel oder
   b) 15%ige Suspension der Glaspartikel in Ethanol

Die Kitkomponenten sind stabil und können bei Raumtemperatur gelagert werden. Nach Auflösung der Proteinase K in Wasser sollte die Lösung aliquotiert und bei -20°C aufbewahrt werden. Die eingefrorene Lösung ist für 12 Monate stabil.

### Standardprotokoll

1. 200 µl Probe werden in ein 2 ml Reaktionsgefäß gegeben und mit 200 µl Bindepuffer und 40 µl Proteinase K-Lösung versetzt. Anschließend wird für 10 min inkubiert. Die Inkubation erfolgt vorzugsweise bei Raumtemperatur. Unter bestimmten Umständen kann die Inkubationstemperatur jedoch auch auf bis zu 70°C erhöht werden.
2. Nach der Inkubation werden 200 µl Isopropanol und eine MGP Tablette (oder alternativ 200 µl MGP Suspension) zugegeben und für 5 min bei Raumtemperatur inkubiert.
3. Das Reaktionsgefäß wird in einen Magnetpartikelseparator (Boehringer Mannheim, Kat. No. 1 641 794) gegeben und für etwa 1 min separiert.
4. Der Überstand wird verworfen und die Reaktionsgefäße werden aus dem MP-Seperator entnommen.
5. Nach Zugabe von 500 µl Waschpuffer wird der Inhalt des Reaktionsgefäßes gemischt und erneut in den MP-Separator für etwa 1 min gegeben.
6. Der Überstand wird verworfen. Schritt 5 wird dreimal widerholt. Nach dem letzten Waschvorgang wird der restliche Waschpuffer vollständig entfernt.
7. Zur Elution werden 100 µl gegebenenfalls auf 70°C vorgewärmter Elutionspuffer zugegeben. Dann wird gemischt und für 5 Minuten bei Raumtemperatur inkubiert. Die Probe wird in den MP-Separator gegeben und der Überstand in ein sauberes Reaktionsgefäß überführt.
8. Die so erhaltenen Nukleinsäuren, z.B. DNA, sind stabil und können anschließend direkt weiterverarbeitet oder bei 4°C aufbewahrt werden.

Das obige Protokoll kann auch entsprechend bei Mikrotiterplatten, z.B. Tieflochmikrotiterplatten (z.B. Ritter, H.J. Bioanalytic), verwendet werden.

### 3. Chlamydia trachomates DNA-Nachweis durch PCR

### 3.1 Manuelles Standardprotokoll zur Probenvorbereitung

200 µl einer Urinprobe und 240 µl Bindepuffer/Proteinase K-Lösung (5:1) werden in ein 2 ml Reaktionsgefäß pipettiert, einem Vortexmischen unterzogen und bei 70°C für 10 min inkubiert. Dann wird die Probe für 5 min auf Raumtemperatur abgekühlt.

Zur Probe werden 200 µl isopropanolische MGP-Lösung pipettiert. Unmittelbar anschließend erfolgt Vortexmischen. Die Probe wird dann für 15 min auf einem Mischer, z.B. Thermomischer 5436 (Eppendorf), inkubiert.

Die MGP werden durch Überführung der Probe in einen Magnetseparator konzentriert. Nach einer Minute wird der Überstand vollständig abpipettiert.

Es werden 0,5 ml Waschpuffer zu den MGPs pipettiert. Die Probe wird einem Vortexmischen unterzogen und dann in den Magnetseparator überführt. Der Überstand wird nach 1 min abpipettiert. Die Waschprozedur wird noch zweimal wiederholt.

Den MGP werden 200 ml Elutionspuffer zugesetzt. Die Probe wird 10 min bei 70°C auf einem Thermomischer bei 1400 RPM inkubiert. Kondenswasser wird durch kurze Zentrifugation gesammelt. Die Probe wird in den Magnetseparator überführt und nach 1 min 180 µl Eluat abgenommen. Das Eluat wird in ein neues Reaktionsgefäß pipettiert und bei 4°C (bei einer Aufbewahrungsdauer < 24 h) oder bei - 20°C (bei längerer Aufbewahrungsdauer) aufbewahrt.

Für die PCR werden 50 µl Eluat eingesetzt. Die Auswertung erfolgt durch Elektrochemilumineszenz.

### 3.2 Protokoll für ein semiautomatisiertes Verfahren

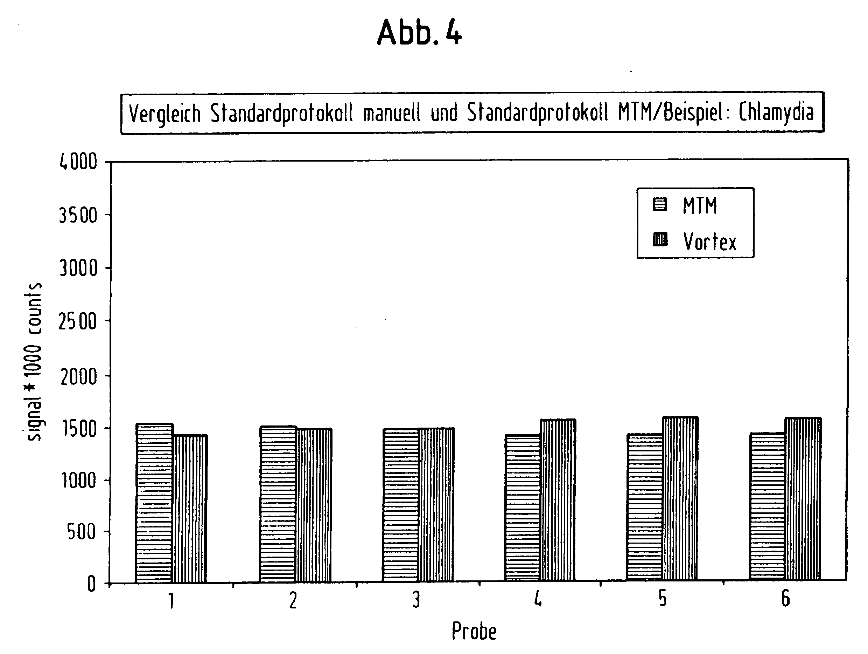

Statt des in 3.1 beschriebenen Vortexmischens und der Temperierung auf einem Thermoblock wird ein semiautomatisiertes Verfahren durchgeführt, bei dem das Mischen und die Temperierung auf einem Misch- und Temperiermodul erfolgt. Abbildung 4 zeigt einen Vergleich der Bestimmung von Chlamydien (Probe: 100 Elementarantikörper pro 100 ml Urin; Sechsfachbestimmung) zwischen dem manuellen Standardprotokoll (Vortex) und dem semiautomatisierten Verfahren (MTM). Es ist ersichtlich, daß durch die Automatisierung keine Beeinträchtigung der Sensitivität erfolgt.

### 3.3 Semiautomatisiertes Protokoll bei Raumtemperatur

Die Probenvorbereitung erfolgt wie unter Punkt 3.2 beschrieben. Die Lyse und die Elution werden jedoch bei Raumtemperatur durchgeführt.

### 3.4 Semiautomatisiertes Probenvorbereitungsprotokoll bei Raumtemperatur mit anschließender Nachbehandlung des Eluats

Die Probenvorbereitung erfolgt wie unter Punkt 3.3 beschrieben. Nach der Elution erfolgt eine Inkubation für 10 min bei 70°C.

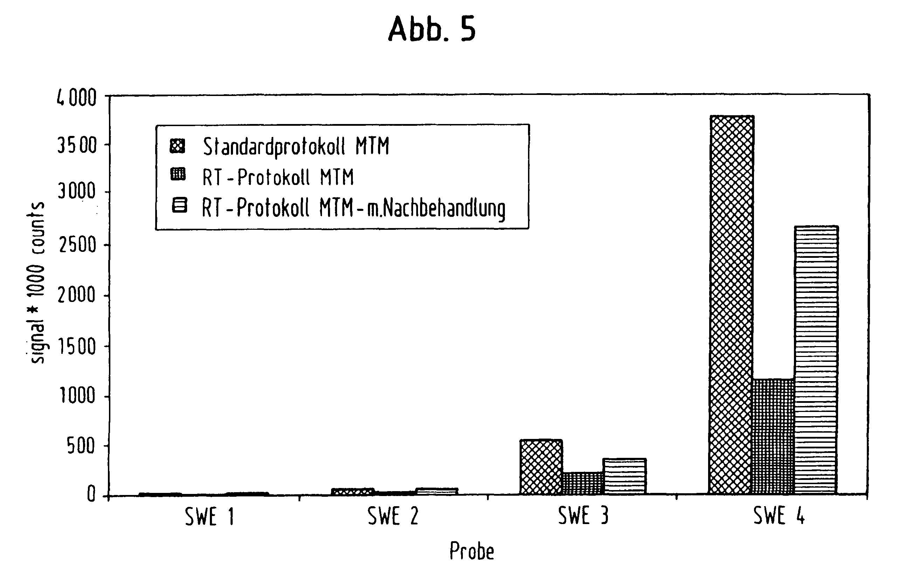

Abbildung 5 zeigt einen Vergleich der Chlamydienbestimmung (Proben: SWE1, O Chlamydia-Elementarantikörper (EAK) pro ml Urin, SWE 2: 10 EAK, SWE 3:100 EAK und SWE 4:1000 EAK jeweils pro ml Urin) zwischen den in den Punkten 3.2, 3.3 und 3.4 beschriebenen Probenvorbereitungsprotokollen. Es ist ersichtlich, daß für die Bestimmung von Chlamydien das Standardprotokoll gegenüber einer Probenvorbereitung bei Raumtemperatur (RT-Protokoll MTM) sensitiver ist. Die Ergebnisse der Probenvorbereitung bei Raumtemperatur und anschließender Nachbehandlung des Eluats (RT-Protokoll MTM mit Nachbehandlung) zeigen jedoch, daß dieser Effekt größtenteils kompensiert werden kann. Überraschenderweise ist daher während der Probenvorbereitung selbst kein Temperaturschritt erforderlich.

Durch diese Erkenntnis läßt sich das Probenvorbereitungsverfahren entscheidend vereinfachen, denn die Schritte der Lyse, der Adsorption, des Waschens und der Elution können bei Temperaturen ≤ 40°c erfolgen, was eine Automatisierung vereinfacht, da keine Deckelgegenheizung und Temperaturregulierung erforderlich ist.

### 4. HIV-RNA Nachweis durch PCR

### 4.1 Manuelles Standardprotokoll zur Probenvorbereitung

Geforenes Plasma wird 5 min bei 37°C aufgetaut und zur weiteren Verarbeitung auf Eis gekühlt.

In ein 1,5 ml Sarstedt-Reaktionsgefäß werden 50 µl einer Proteinase K Lösung (25 mg/ml) pipettiert. Dazu werden 250 µl Probe gegeben und im Vortex gemischt. Dann werden 300 µl Lysepuffer zugegeben und erneut im Vortex gemischt.

Es wird 10 min bei Raumtemperatur auf einem Eppendorfmischer bei 13.000 RPM inkubiert. Dann werden 300 µl einer MGP Suspension (6 mg/ml MGP in Isopropanol) zugegeben, im Vortex gemischt und 20 min bei Raumtemperatur bei fortgesetztem Mischen inkubiert. Die MGP werden auf einem Magnetseperator abgetrennt und der Überstand vollständig entfernt.

Zu den MGP werden 750 µl Waschpuffer gegeben. Die MGP werden resuspendiert und wie zuvor beschrieben abgetrennt. Die Waschprozedur wird viermal wiederholt, wobei am Ende der Waschpuffer sorgfältig entfernt wird.

Dann werden 100 µl Elutionspuffer zugegeben und die MGP resuspendiert. Nach 15minütiger Inkubation bei 80°C auf einem Eppendorf Thermomischer (13.000 RPM) werden 90 µl Eluat in eine neues Reaktionsgefäß überführt. Für die anschließende HIV-Bestimmung durch RT-PCR werden 40 µl Eluat verwendet.

### 4.2 Semiautomatisiertes Standardprotokoll für die Probenvorbereitung

Die Probenvorbereitung erfolgt wie unter Punkt 4.1 beschrieben, abgesehen davon, daß das Mischen und die Temperierung auf einem Misch- und Temperiermodul erfolgte.

### 4.3 Semiautomatisiertes Protokoll bei Raumtemperatur

Die Probenvorbereitung erfolgt im wesentlichen wie unter Punkt 4.2 beschrieben, außer daß alle Schritte bei Raumtemperatur durchgeführt werden. Die Inkubationsdauer für Lyse, Adsorption und Elution beträgt jeweils 15 min.

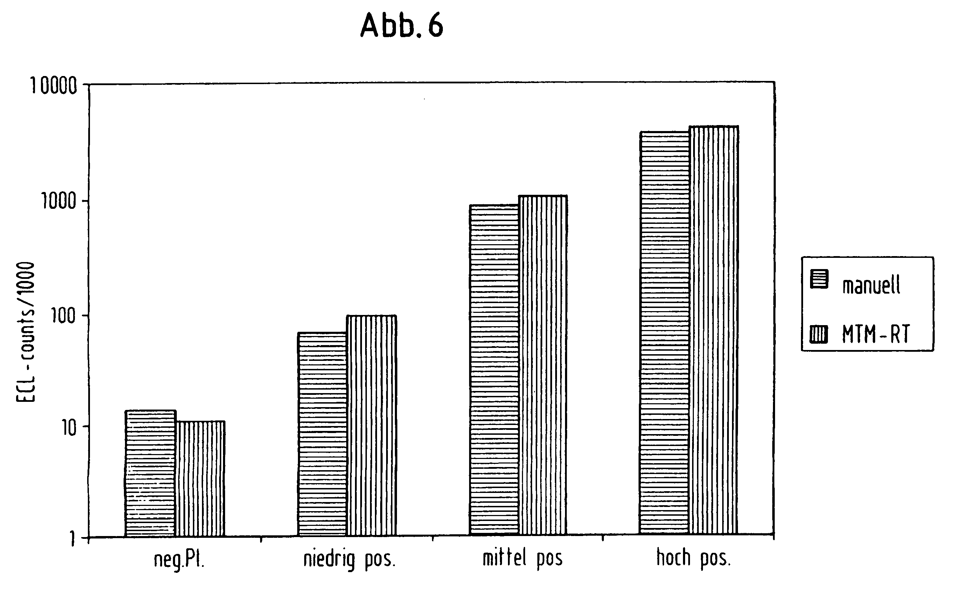

Aus Abbildung 6 ist ersichtlich, daß durch die Automatisierung und die Probenvorbereitung bei Raumtemperatur (RT-Protokoll MTM) keine Beeinträchtigung der Sensitivität gegenüber dem Standardprotokoll bei manueller Probenvorbereitung (manuell) erfolgt. Sowohl bei negativen, niedrigpositiven, mittelpositiven und hochpositiven Plasmen werden reproduzierbare Ergebnisse erhalten.

## Patentansprüche

1. Verfahren zur Isolierung eines Nukleinsäure-Analyten aus einer biologischen Probe, umfassend die Schritte:
(a) Aufschließen der Probe in einem Reaktionsgefäß,
(b) Zugeben von magnetischen Glaspartikel in Form einer alkoholischen Suspension,
(c) Inkubieren unter Bedingungen, bei denen der Analyt an die Glaspartikel bindet,
(d) Entfernen nichtgebundener Probenbestandteile aus dem Reaktionsgefäß.
(e) Inkubieren unter Bedingungen, bei denen der Analyt von den Glaspartikeln eluiert wird, und
(f) Abtrennen des Eluats von den Glaspartikeln,
wobei viele oder alle Schritte bei im wesentlichen der gleichen Temperatur durchgeführt werden.

2. Verfahren nach Anspruch 1,
**dadurch gekennzeichnet,**
**daß** Schritt (a) die Zugabe einer Protease und eines Denaturierungspuffers umfaßt.

3. Verfahren nach Anspruch 2,
**dadurch gekennzeichnet,**
**daß** man Proteinase K als Protease verwendet.

4. Verfahren nach Anspruch 2,
**dadurch gekennzeichnet,**
**daß** man einen Denaturierungspuffer, der ein Guanidiniumsalz, insbesondere Guanidiniumhydrochlorid oder/und Guanidiniumthiocyanat enthält, verwendet.

5. Verfahren nach einem der vorhergehenden Ansprüche,
**dadurch gekennzeichnet,**
**daß** man zumindest die Schritte (a) bis (e) bei einer im wesentlichen gleichen Temperatur durchgeführt werden, die im Bereich von Raumtemperatur bis 40°C liegt.

6. Verfahren nach einem der Ansprüche 1 bis 5,
**dadurch gekennzeichnet,**
**dass** die alkoholische Suspension eine Konzentration von etwa 5 bis 20 mg/ml hat.

7. Verfahren nach Anspruch 5 oder 6,
**dadurch gekennzeichnet,**
**daß** man Glaspartikel verwendet, deren Glasphase SiO₂, B₂O₃ und Na₂O oder SiO₂, B₂O₃, Al₂O₃, CaO und K₂O enthält.

8. Verfahren nach einem der vorhergehenden Ansprüche,
**dadurch gekennzeichnet,**
**daß** die Glaspartikel einer Menge zugegeben werden, die um höchstens 50 % über der Menge liegt, die zur quantitativen Bindung des in der Probe vorliegenden Analyten benötigt wird.

9. Verfahren nach einem der vorhergehenden Ansprüche,
**dadurch gekennzeichnet,**
**daß** zumindest während der Schritte (c), (d) oder/und (e) ein kontinuierliches oder intervallartiges Mischen ohne Zusatz externer Mittel erfolgt.

10. Verfahren nach Anspruch 9,
**dadurch gekennzeichnet,**
**daß** das Mischen durch Drehung des Reaktionsgefäßes um seine Längsachse erfolgt.

11. Verfahren nach Anspruch 9 oder 10, ,
**dadurch gekennzeichnet,**
**daß** die Dauer zur Durchführung der Schritte (c) oder/und (e) jeweils maximal 20 min beträgt.

12. Verfahren nach einem der vorhergehenden Ansprüche,
**dadurch gekennzeichnet,**
**daß** Schritt (d) ein gegebenenfalls mehrmaliges Zugeben und Absaugen eines Waschpuffers umfaßt.

13. Verfahren nach Anspruch 12,
**dadurch gekennzeichnet,**
**daß** man einen Waschpuffer mit einem Gehalt von mindestens 50 % (v/v) eines mit Wasser mischbaren organischen Lösungsmittels verwendet.

14. Verfahren nach einem der vorhergehenden Ansprüche,
**dadurch gekennzeichnet,**
**daß** in Schritt (e) zusätzliche Reagenzien wie etwa Enzyme zugesetzt werden.

15. Verfahren nach einem der vorhergehenden Ansprüche,
**dadurch gekennzeichnet,**
**daß** in Schritt (e) zur Elution ein Niedrigsalzpuffer verwendet wird.

16. Verfahren nach einem der Ansprüche 1 bis 14,
**dadurch gekennzeichnet,**
**daß** in Schritt (e) zur Elution ein Nukleinsäureamplifikations-Mastermix zugesetzt wird.

17. Verfahren nach einem der Ansprüche 1 bis 16,
**dadurch gekennzeichnet,**
**daß** die Temperatur im Bereich von 18° C bis 32° C liegt.

18. Verfahren nach einem der vorhergehenden Ansprüche,
**dadurch gekennzeichnet,**
**daß** nach Schritt (f) ein Nachbehandlungsschritt bei erhöhter Temperatur erfolgt.

19. Verfahren nach Anspruch 18,
**dadurch gekennzeichnet**
**daß** die erhöhte Temperatur im Bereich von mehr als 40° C bis 95° C liegt.

20. Verfahren zur Isolierung eines Nukleinsäure-Analyten aus einer biologischen Probe, umfassend die Schritte:
(a) Aufschließen der Probe in einem Reaktionsgefäß,
(b) Zugeben von magnetischen Glaspartikel in Form einer alkoholischen Suspension,
(c) Inkubieren unter Bedingungen , bei denen der Analyt an die Glaspartikel bindet,
(d) Abtrennen der nichtgebundenen Probenbestandteile von den Glaspartikeln,
(e) Inkubieren unter Bedingungen, bei denen der Analyt von den Glaspartikeln eluiert wird, und
(f) Abtrennen des Eluats von den Glaspartikeln,
wobei viele oder alle Schritte bei im wesentlichen der gleichen Temperatur durchgeführt werden.

21. Verfahren nach Anspruch 20,
wobei zumindest die Schritte (a) bis (e) bei im wesentlichen der gleichen Temperatur durchgeführt werden, die im Bereich von Raumtemperatur bis 40° C liegt.

22. Verfahren nach einem der vorhergehenden Ansprüche,
**dadurch gekennzeichnet,**
**daß** die Durchführung des Verfahrens in einer automatisierten Vorrichtung erfolgt.

23. Verfahren nach Anspruch 1 bis 22,
**dadurch gekennzeichnet,**
**daß** die Schritte (a) bis (e) in einem einzigen Reaktionsgefäß durchgeführt werden.

24. Reagenzienkit zur Isolierung eines Nukleinsäure-Analyten, insbesondere zur Durchführung des Verfahrens nach einem der Ansprüche 1 bis 23,
umfassend
(a) eine Protease,
(b) einen Probenaufschlußpuffer,
(c) einen Waschpuffer,
(d) einen Elutionspuffer und
(e) eine alkoholische Suspension von magnetischen Glaspartikeln.

## Claims

1. Process for the isolation of a nucleic acid analyte from a biological sample comprising the steps:
(a) lysing the sample in a reaction vessel,
(b) adding magnetic glass particles in the form of an alcoholic suspension,
(c) incubating under conditions in which the analyte binds to the glass particles,
(d) removing non-bound sample components from the reaction vessel,
(e) incubating under conditions in which the analyte is eluted from the glass particles and
(f) separating the eluate from the glass particles
wherein many or all steps are carried out at essentially the same temperature.

2. Process according to claim 1,
**characterized in that**
step (a) comprises adding a protease and a denaturing buffer.

3. Process according to claim 2,
**characterized in that**
proteinase K is used as the protease.

4. Process according to claim 2,
**characterized in that**
a denaturing buffer is used which contains a guanidinium salt, in particular guanidinium hydrochloride or/and guanidinium thiocyanate.

5. Process according to one of the previous claims,
**characterized in that**
at least steps (a) to (e) are carried out at an essentially identical temperature which is in the range from room temperature to 40°C.

6. Process according to one of the claims 1 to 5,
**characterized in that**
the alcoholic suspension has a concentration of about 5 to 20 mg/ml.

7. Process according to claim 5 or 6,
**characterized in that**
glass particles are used whose glass phase contains SiO₂, B₂O₃ and Na₂O or SiO₂ B₂O₃, Al₂O₃, CaO and K₂O.

8. Process according to one of the previous claims,
**characterized in that**
the glass particles are added in an amount that is at most 50 % more than the amount that is required to quantitatively bind the analyte present in the sample.

9. Process according to one of the previous claims,
**characterized in that**
a continuous or intermittent mixing without adding external means is carried out at least during steps (c), (d) or/and (e).

10. Process according to claim 9,
**characterized in that**
the mixing is achieved by rotating the reaction vessel around its longitudinal axis.

11. Process according to claims 9 or 10,
**characterized in that**
the maximum period for carrying out steps (c) or/and (e) is 20 min in each case.

12. Process according to one of the previous claims,
**characterized in that**
step (d) comprises adding and aspirating a wash buffer which is optionally repeated several times.

13. Process according to claim 12,
**characterized in that**
a wash buffer is used with a content of at least 50 % (v/v) of an organic solvent that is miscible with water.

14. Process according to one of the previous claims,
**characterized in that**
additional reagents such as enzymes are added in step (e).

15. Process according to one of the previous claims,
**characterized in that**
a low salt buffer is used for elution in step (e).

16. Process according to one of the claims 1 to 14,
**characterized in that**
a nucleic acid amplification master mix is added for elution in step (e).

17. Process according to one of the claims 1 to 16,
**characterized in that**
the temperature is in the range of 18°C to 32°C.

18. Process according to one of the previous claims,
**characterized in that**
an aftertreatment step at an elevated temperature is carried out after step (f).

19. Process according to claim 18,
**characterized in that**
the elevated temperature is in the range from more than 40°C to 95°C.

20. Process for the isolation of a nucleic acid analyte from a biological sample comprising the steps:
(a) lysing the sample in a reaction vessel,
(b) adding magnetic glass particles in the form of an alcoholic suspension,
(c) incubating under conditions in which the analyte binds to the glass particles,
(d) separating non-bound sample components from the glass particles,
(e) incubating under conditions in which the analyte is eluted from the glass particles and
(f) separating the eluate from the glass particles
wherein many or all steps are carried out at essentially the same temperature.

21. Process according to claim 20,
**characterized in that**
at least the steps (a) to (e) are carried out at essentially the same temperature which is in the range from room temperature to 40°C.

22. Process according to one of the previous claims,
**characterized in that**
the process is carried out in an automated device.

23. Process according to claim 1 to 22,
**characterized in that**
steps (a) to (e) are carried out in a single reaction vessel.

24. Reagent kit for isolating a nucleic acid analyte, in particular to carry out the process according to one of the claims 1 to 23,
comprising
(a) a protease,
(b) a sample lysing buffer,
(c) a wash buffer,
(d) an elution buffer and
(e) an alcoholic suspension of magnetic glass particles.

## Revendications

1. Procédé pour isoler un analyte de type acide nucléique d'un échantillon biologique, comprenant les étapes :
(a) de digestion de l'échantillon dans un récipient de réaction,
(b) d'addition de particules de verre magnétiques sous forme d'une suspension alcoolique,
(c) d'incubation dans des conditions dans lesquelles l'analyte se lie aux particules de verre,
(d) d'élimination des constituants de l'échantillon qui n'ont pas été liés du récipient de réaction,
(e) d'incubation dans des conditions dans lesquelles l'analyte est élué des particules de verre et
(f) de séparation de l'éluat des particules de verre;
un grand nombre ou toutes les étapes étant réalisées essentiellement à la même température.

2. Procédé selon la revendication 1, **caractérisé en ce que** l'étape (a) comprend l'addition d'une protéase et d'un tampon de dénaturation.

3. Procédé selon la revendication 2, **caractérisé en ce qu'**on utilise de la protéinase K comme protéase.

4. Procédé selon la revendication 2, **caractérisé en ce qu'**on utilise un tampon de dénaturation qui contient un sel de guanidinium, en particulier un chlorhydrate de guanidinium ou/et un thiocyanate de guanidinium.

5. Procédé selon l'une quelconque des revendications précédentes, **caractérisé en ce qu'**au moins les étapes (a) à (e) sont réalisées à une température essentiellement identique qui se situe dans la plage de la température ambiante à 40°C.

6. Procédé selon l'une quelconque des revendications 1 à 5, **caractérisé en ce que** la suspension alcoolique présente une concentration d'environ 5 à 20 mg/ml.

7. Procédé selon la revendication 5 ou 6, **caractérisé en ce qu'**on utilise des particules de verre dont la phase de verre contient du SiO₂, du B₂O₃ et du Na₂O ou du SiO₂, du B₂O₃, de l'Al₂O₃, du CaO et du K₂O.

8. Procédé selon l'une quelconque des revendications précédentes, **caractérisé en ce que** les particules de verre sont ajoutées en une quantité qui est supérieure d'au plus 50% à la quantité nécessaire pour la liaison quantitative de l'analyte se trouvant dans l'échantillon.

9. Procédé selon l'une quelconque des revendications précédentes, **caractérisé en ce qu'**au moins pendant les étapes (c), (d) ou/et (e), on réalise un mélange en continu ou par intervalles sans addition d'agents externes.

10. Procédé selon la revendication 9, **caractérisé en ce que** le mélange est réalisé par rotation du récipient de réaction autour de son axe longitudinal.

11. Procédé selon la revendication 9 ou 10, **caractérisé en ce que** la durée pour réaliser les étapes (c) ou/et (e) est à chaque fois d'au maximum 20 min.

12. Procédé selon l'une quelconque des revendications précédentes, **caractérisé en ce que** l'étape (d) comprend une addition et une aspiration, le cas échéant multiple, d'un tampon de lavage.

13. Procédé selon la revendication 12, **caractérisé en ce qu'**on utilise un tampon de lavage présentant une teneur d'au moins 50% (VN) d'un solvant organique miscible à l'eau.

14. Procédé selon l'une quelconque des revendications précédentes, **caractérisé en ce qu'**on utilise dans l'étape (e) des réactifs supplémentaires tels que par exemple des enzymes.

15. Procédé selon l'une quelconque des revendications précédentes, **caractérisé en ce qu'**on utilise dans l'étape (e) un tampon faiblement salin pour l'élution.

16. Procédé selon l'une quelconque des revendications 1 à 14, **caractérisé en ce qu'**on ajoute dans l'étape (e), pour l'élution, un mélange-maître d'amplification d'acides nucléiques.

17. Procédé selon l'une quelconque des revendications 1 à 16, **caractérisé en ce que** la température se situe dans la plage de 18°C à 32°C.

18. Procédé selon l'une quelconque des revendications précédentes, **caractérisé en ce qu'**une étape de post-traitement à température élevée a lieu après l'étape (f).

19. Procédé selon la revendication 18, **caractérisé en ce que** la température élevée se situe dans la plage de plus de 40°C à 95°C.

20. Procédé pour isoler un analyte de type acide nucléique d'un échantillon biologique, comprenant les étapes :
(a) de digestion de l'échantillon dans un récipient de réaction,
(b) d'addition de particules de verre magnétiques sous forme d'une suspension alcoolique,
(c) d'incubation dans des conditions dans lesquelles l'analyte se lie aux particules de verre,
(d) de séparation des constituants de l'échantillon qui n'ont pas été liés des particules de verre,
(e) d'incubation dans des conditions dans lesquelles l'analyte est élué des particules de verre et
(f) de séparation de l'éluat des particules de verre,
un grand nombre ou toutes les étapes étant réalisées essentiellement à la même température.

21. Procédé selon la revendication 20, **caractérisé en ce qu'**au moins les étapes (a) à (e) sont réalisées à une température essentiellement identique qui se situe dans la plage de la température ambiante à 40°C.

22. Procédé selon l'une quelconque des revendications précédentes, **caractérisé en ce que** la réalisation du procédé a lieu dans un dispositif automatisé.

23. Procédé selon la revendication 1 à 22, **caractérisé en ce que** les étapes (a) à (e) sont réalisées dans un seul récipient de réaction.

24. Kit de réactifs pour isoler un analyte de type acide nucléique, en particulier pour réaliser le procédé selon l'une quelconque des revendications 1 à 23, comprenant
(a) une protéase,
(b) un tampon de digestion de l'échantillon,
(c) un tampon de lavage,
(d) un tampon d'élution et
(e) une suspension alcoolique de particules de verre magnétiques.
